# EUROPÄISCHE PATENTSCHRIFT

(11) **EP 1 593 963 B1**
(45) Veröffentlichungstag und Bekanntmachung des Hinweises auf die Patenterteilung: **13.11.2013**
(21) Anmeldenummer: 05007711.4
(22) Anmeldetag: 08.04.2005
(51) Int. Cl.: G01N 27/411, G01N 33/20

(54) **Messeinrichtung zur Bestimmung der Sauerstoffaktivität in Metall- oder Schlackeschmelzen**
Measurement set-up to determine the oxygen activity in metallic or slag melts
Appareil de mesure pour la détermination de l'activité d'oxygène dans du metal ou des scories fondus

(30) Priorität: 05.05.2004 DE 102004022763
(43) Veröffentlichungstag der Anmeldung: 09.11.2005
(73) Patentinhaber: Heraeus Electro-Nite International N.V., 3530 Houthalen (BE)
(72) Erfinder: Habets, Danny, 3600 Genk (BE)
(74) Vertreter: Kühn, Hans-Christian

(56) Entgegenhaltungen:
- EP-A- 0 295 112
- US-A- 4 451 350
- PATENT ABSTRACTS OF JAPAN Bd. 009, Nr. 177 (P-375), 23. Juli 1985 (1985-07-23) & JP 60 052763 A (SUMITOMO ARUMINIUMU SEIREN KK), 26. März 1985 (1985-03-26)
- IWASE M: "Rapid determination of silicon activities in hot metal by means of solid state electrochemical sensors equipped with an auxiliary electrode" SCANDINAVIAN JOURNAL OF METALLURGY, Bd. 17, 1988, Seiten 50-56, XP009052218
- PATENT ABSTRACTS OF JAPAN Bd. 009, Nr. 227 (P-388), 13. September 1985 (1985-09-13) & JP 60 085361 A (KOBE SEIKOSHO KK), 14. Mai 1985 (1985-05-14)

## Beschreibung

Die Erfindung betrifft eine Messeinrichtung zur Bestimmung der Sauerstoffaktivität in Metall- oder Schlackeschmelzen mit einem an einem Ende eines Trägerrohrs angeordneten Messkopf, an dem eine elektrochemische Messzelle angeordnet ist, wobei die elektrochemische Messzelle ein einseitig geschlossenes Festelektrolytröhrchen aufweist, das an seinem geschlossenen Ende ein Referenzmaterial und eine Elektrode enthält, wobei die Elektrode aus dem entgegengesetzten Ende des Festelektrolytröhrchens hinausragt. Des weiteren betrifft die Erfindung ein Festelektrolytröhrchen für eine elektrochemische Messzelle.

Derartige Messeinrichtungen sind beispielsweise aus DE 31 52 318 C2 bekannt. Der hier beschriebene Sensor dient zur Messung der Konzentration von Sauerstoff in geschmolzenem Metall. Ähnliche Messeinrichtungen sind auch aus US 3,578,578, DE 28 10 134 A1 oder DE 26 00 103 C2 bekannt. In US 4,657,641 ist ein Sensor offenbart, der eine Beschichtung aus Zirkoniumsilikat und Zirkoniumdioxid aufweist. Aus EP-A-0 295 112 ist eine gattungsgemäße Messeinrichtung bekannt, bei der das Festelektrolytröhrchen eine Schicht aus einer Mischung von Oxiden, Fluoriden und einem organischen Bindemittel aufweist.

Es besteht der Bedarf, neben Sauerstoff auch andere in Metallschmelzen enthaltene Stoffe zu messen. Der Erfindung liegt daher die Aufgabe zu Grunde, eine einfache Messeinrichtung sowie ein entsprechendes Festelektrolytröhrchen anzugeben, mit dem neben dem Sauerstoffgehalt auch die Konzentration weiterer Elemente ermittelt werden kann.

Die Aufgabe wird dadurch gelöst, dass das Festelektrolytröhrchen an seiner äußeren Oberfläche eine Beschichtung aus einer Mischung aus Zirkoniumsilikat mit MgF₂ aufweist wobei die Schichtdicke 10 bis 50 µm beträgt und die Schicht plasma- oder flammgespritzt ist. Es hat sich gezeigt, dass damit die Ermittlung beispielsweise der Konzentration von Silizium oder Kohlenstoff in Schmelzen möglich ist. Der Effekt wird so erklärt, dass beispielsweise des in flüssigem Metall befindliche Silizium mit dem SiO₂ aus dem Zirkoniumsilikat reagiert, wobei in einer Gleichgewichtsreaktion Sauerstoff als Reaktionsprodukt erhalten wird und die Änderung der Sauerstoffaktivität an der Oberfläche des Festelektrolyten gemessen und mit dem Silizium korreliert wird. Die Messeinrichtung kann in Metall- oder Schlackeschmeizen, insbesondere in Stahl- oder Eisenschmelzen, zur Messung der Konzentration von Silizium oder Kohlenstoff verwendet werden. Dadurch ergibt sich eine schnelle Messung. Vorteilhaft ist es, wenn die Messeinrichtung neben der elektrochemischen Messzelle einen Temperatursensor, beispielsweise ein Thermoelement aufweist, so dass auch die Temperatur der Metallschmelze gemessen wer den kann. Kohlenstoff kann aus dem Silizium-Kohlenstoff-Temperaturgleichgewicht des flüssigen Metalls berechnet werden.

Durch die erfindungsgemäße Messeinrichtung kann eine Proben analyse im Labor vermieden werden, so dass im Produktionsprozess eine erhebliche Zeiteinsparung und damit eine bessere und schnellere Beeinflussung des Produktionsprozesses erzielt werden kann.

Vorteilhafte Ausgestaltungen der Erfindung sind in den Unteransprüchen angegeben. Es ist von Vorteil, dass die Schichtdicke etwa 30 µm beträgt. Dabei reicht eine dickere Schicht bei höheren Einsatztemperaturen (ab etwa 1500°C), beispielsweise vor einer Entschwefelungsbehandlung, aus. Die Antwortzeit ist dabei recht kurz. Bei niedrigeren Einsatztemperaturen (bis etwa 1400°C), beispielsweise nach der Entschwefelungsbehandlung, ist eine dünnere Schicht erforderlich. Die Antwortzeit ist dann etwas größer. Vorteilhaft werden 2 bis 10 Gew.% Fluorid, insbesondere ca. 3 bis 4 Gew.-% Fluorid verwendet (bezogen auf die Beschichtung). Bei hohem Fluorid-Gehalten ist die Antwortzeit bei niedrigeren Schmelztemperaturen kürzer, da das Gleichgewicht schneller erreicht ist. Die Beschichtung kann mittels Plasmaspritzen oder Flammspritzen sehr gleichmäßig hergestellt sein.

Das Festelektrolytröhrchen ist vorteilhafterweise stabilisiertes ZrO₂. Die Schicht kann die äußere Oberfläche des Festelektrolytröhrchens auch nur teilweise bedecken, wobei mindestens die Oberfläche in dem Bereich des Röhrchens beschichtet sein sollte, in dem die Bezugsmasse angeordnet ist.

Nachfolgend wird ein Ausführungsbeispiel der Erfindung anhand einer Zeichnung erläutert. In der Zeichnung zeigt:
- Figur 1: eine Messeinrichtung und
- Figur 2: den Querschnitt durch ein Festelektrolytröhrchen.

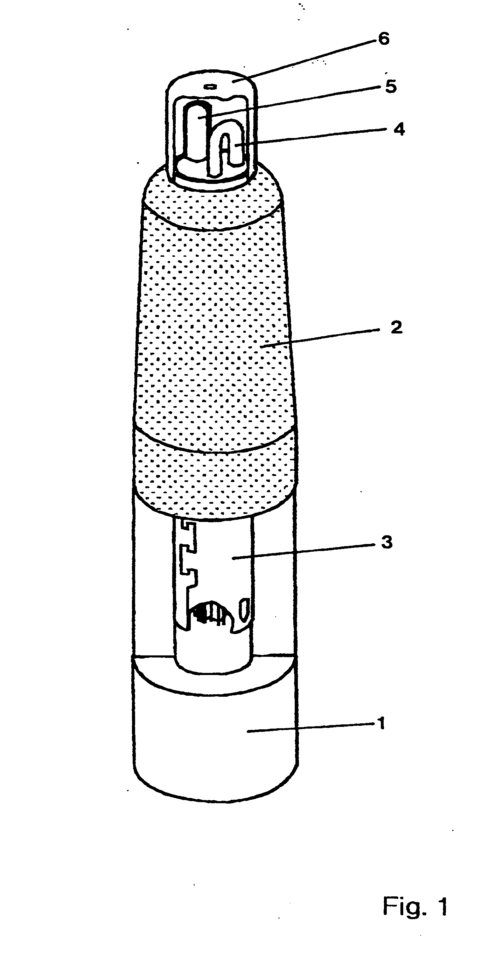

Die Messeinrichtung weist ein Trägerrohr 1 auf, in dem der Messkopf 2 gehalten ist, wobei der Messkopf 2 innerhalb des Trägerrohrs 1 mittels eines Köntaktstückes 3 mit einer Zuleitung zu Mess- und Auswertegeräten kontaktiert. Das Trägerrohr 1 ist in der Figur 1 nur ansatzweise dargestellt.

Am Eintauchende des Messkopfes ist neben einem Thermoelement 4 ein Festelektrolytröhrchen 5 angeordnet. Thermoelement 4 und Festelektrolytröhrchen 5 sind von einer Schutzkappe 6 umgeben und vor bzw. während des Eintauchens des Messkopfes in die Schmelze, insbesondere eine Eisen- oder Stahlschmelze, geschützt.

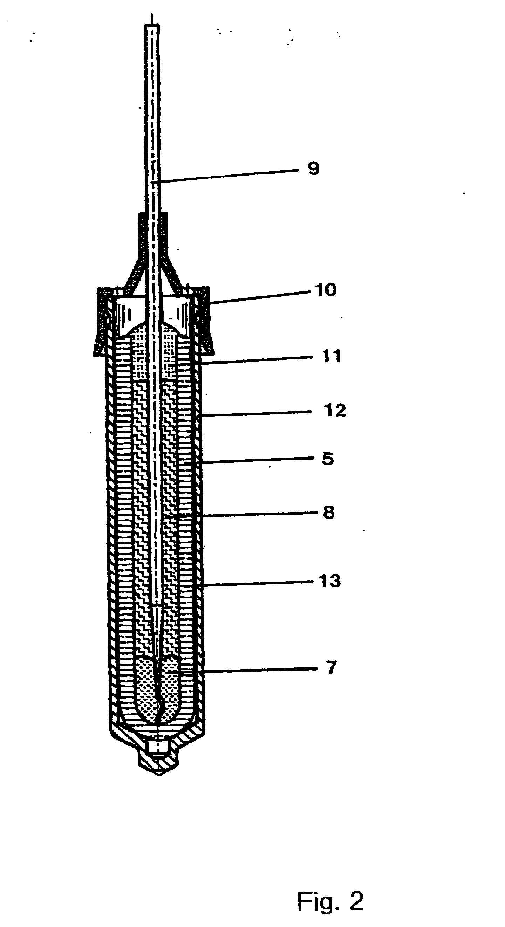

In Figur 2 ist das Festelektrolytröhrchen 5 im Schnitt dargestellt. Es ist aus stabilisiertem Zirkondioxid hergestellt und weist in seinem Inneren als Referenzmaterial 7 eine Mischung aus Molybdän und Molybdändioxid, aus Chrom und Chromdioxid oder aus Nickel und Nickeloxid auf. Das darüber angeordnete Füllmaterial 8 ist beispielsweise Aluminiumoxid. In dem Festelektrolytröhrchen 5 ist zentrisch ein Molybdänstab als Elektrode 9 angeordnet. Die Elektrode 9 ragt aus dem offenen Ende des Festelektrolytröhrchens 5 heraus. Dieses offene Ende ist von einer Kappe 10 verschlossen, wobei das Füllmaterial 8 an seinem oberen Ende von einem gasdurchlässigen Zement 11 gehalten ist. Das Festelektrolytröhrchen 5 ist von einer Stahlkappe 12 umgeben, die das Röhrchen während des Eintauchens in die Metallschmelze zusätzlich schützt. Es schmilzt dann und gibt die auf dem Festelektrolytröhrchen 5 angeordnete Beschichtung 13 frei. Die Beschichtung ist vorzugsweise eine Mischung aus Zirkoniumsilikat und ca. 3 bis 4 Gew.-% Magnesiumfluorid. Sie ist etwa 30 µm dick und plasmagespritzt.

In der Schmelze reagiert SiO₂ mit dem Si der Schmelze, wobei in einer Gleichgewichtsreaktion Sauerstoff frei wird, dessen Aktivität mit Hilfe des Festelektrolytröhrchens gemessen wird.

## Patentansprüche

1. Messeinrichtung zur Bestimmung der Sauerstoffaktivität in Metall- oder Schlackeschmelzen mit einem an einem Ende eines Trägerrohrs angeordneten Messkopf, an dem eine elektrochemische Messzelle angeordnet ist, wobei die elektrochemische Messzelle ein einseitig geschlossenes Festelektrolytröhrchen aufweist, das an seinem geschlossenen Ende ein Referenzmaterial und eine Elektrode enthält, wobei die Elektrode aus dem entgegengesetzten Ende des Festelektrolytröhrchens hinausragt, **dadurch gekennzeichnet, dass** das Festelektrolytrührchen (5) an seiner äußeren Oberfläche eine Beschichtung (13) aus einer Mischung aus Zirkoniumsilikat mit MgF₂ aufweist, wobei die Beschichtung (13) 10 bis 50 µm dick und plasma- oder flammgespritzt ist.

2. Messeinrichtung nach Anspruch 1, **dadurch gekennzeichnet, dass** die Beschichtung (13) die äußere Oberfläche des Festelekirolytröhrchens (5) nur teilweise bedeckt.

3. Festelektrolytröhrchen für eine elektrochemische Meßzelle mit einem geschlossenen Ende, **dadurch gekennzeichnet, daß** es an seiner äußeren Oberfläche eine Beschichtung (13) aus einer Mischung aus Zirkoniumsilikat mit MgF₂ aufweist, wobei die Beschichtung (13) 10 bis 50 µm dick und plasma- oder flammgespritzt ist.

4. Festelektrolytröhrchen nach Anspruch 3, **dadurch gekennzeichnet, dass** die Beschichtung (13) die äußere Oberfläche nur teilweise bedeckt.

5. Verwendung der Messeinrichtung nach einem der Ansprüche 1 oder 2 zur Bestimmung des Gehaltes von Silizium oder Kohlenstoff in Metall- oder Schlackeschmelzen, insbesondere in Stahl- oder Eisenschmelzen.

## Claims

1. Measuring facility for determining the oxygen activity in melted metal or melted slag, having a measuring head arranged on one end of a support tube, an electrochemical measuring cell being arranged on said measuring head, whereby the electrochemical measuring cell comprises a solid electrolyte tube that is closed on one end and contains a reference material and an electrode on its closed end, whereby the electrode projects from the opposite end of the solid electrolyte tube, **characterised in that** the solid electrolyte tube (5) comprises on its external surface a coating (13) made of a mixture of zirconium silicate and MgF₂, whereby the coating (13) is 10 to 50 µm in thickness and is plasma- or flame-sprayed.

2. Measuring facility according to claim 1, **characterised in that** the coating (13) covers the external surface of the solid electrolyte tube (5) only partly.

3. Solid electrolyte tube for an electrochemical measuring cell having a closed end, **characterised in that** it comprises on its external surface a coating (13) made of a mixture of zirconium silicate and MgF₂, whereby the coating (13) is 10 to 50 µm in thickness and is plasma- or flame-sprayed.

4. Solid electrolyte tube according to claim 3, **characterised in that** the coating (13) covers the external surface only partly.

5. Use of the measuring facility according to any one of the claims 1 or 2 for determining the silicon or carbon content of melted metals or melted slags, in particular of melted steel or melted iron.

## Revendications

1. Dispositif de mesure pour déterminer l'activité d'oxygène dans des masses fondues métalliques ou de scorie comprenant une tête de mesure disposée à une extrémité d'un tuyau de support au niveau de laquelle une cellule de mesure électrochimique est disposée, dans lequel la cellule de mesure électrochimique présente un tube capillaire à électrolyte solide fermé d'un côté qui contient à son extrémité fermée un matériau de référence et une électrode, dans lequel l'électrode dépasse de l'extrémité opposée du tube capillaire à électrolyte solide, **caractérisé en ce que** le tube capillaire à électrolyte solide (5) présente sur sa surface extérieure un revêtement (13) constitué d'un mélange de silicate de zirconium avec MgF₂, dans lequel le revêtement (13) a une épaisseur de 10 à 50 µm et est pulvérisé au plasma ou à la flamme.

2. Dispositif de mesure selon la revendication 1, **caractérisé en ce que** le revêtement (13) ne recouvre que partiellement la surface extérieure du tube capillaire à électrolyte solide (5).

3. Tube capillaire à électrolyte solide pour une cellule de mesure électrochimique avec une extrémité fermée, **caractérisé en ce qu'**il présente sur sa surface extérieure un revêtement (13) constitué d'un mélange de silicate de zirconium avec MgF₂, dans lequel le revêtement (13) a une épaisseur de 10 à 50 µm et est pulvérisé au plasma ou à la flamme.

4. Tube capillaire à électrolyte solide selon la revendication 3, **caractérisé en ce que** le revêtement (13) ne recouvre que partiellement la surface extérieure.

5. Utilisation du dispositif de mesure selon l'une quelconque des revendications 1 ou 2 pour déterminer la teneur en silicium ou carbone dans des masses fondues métalliques ou de scorie, notamment dans des masses fondues d'acier ou de fer.
